# EUROPEAN PATENT SPECIFICATION

(11) **EP 2 053 931 B1**
(45) Date of publication and mention of the grant of the patent: **08.02.2012**
(21) Application number: 07787734.8
(22) Date of filing: 19.07.2007
(51) Int. Cl.: A23L 1/0524, A23L 1/0532, A23L 2/52, A23L 1/29, A23L 2/02, A23L 1/308

(54) **LIQUID SATIETY ENHANCING COMPOSITION**
FLÜSSIGE, DAS VÖLLEGEFÜHL VERSTÄRKENDE ZUSAMMENSETZUNG
COMPOSITION LIQUIDE AUGMENTANT LA SATIÉTÉ

(30) Priority: 24.08.2006 EP 06119434
(43) Date of publication of application: 06.05.2009
(73) Proprietor: Unilever N.V., 3012 AL Rotterdam (NL); Unilever PLC, London EC4Y 0DY (GB)
(72) Inventor: BOERS, Hanny, Margriet, 3133 AT Vlaardingen (NL); STROM, Anna, Helena, Edvardsdotter, 3133 AT Vlaardingen (NL); WISEMAN, Sheila, Ann, 3133 AT Vlaardingen (NL)
(74) Representative: Wurfbain, Gilles L.
(86) International application number: PCT/EP2007/057476
(87) International publication number: WO 2008/022857

(56) References cited:
- EP-A- 1 338 210
- EP-A- 1 588 629
- EP-A1- 1 488 812
- WO-A-03/086360
- WO-A1-03/053169
- WO-A2-02/096219
- WO-A2-03/003849
- GB-A- 2 008 408
- US-A- 5 866 190
- MANCINI F MCHUGH T H: "Fruit-alginate interactions in novel restructured products" NAHRUNG - FOOD, VCH VERLAGSGESELLSCHAFT, WEINHEIM, XX, vol. 44, no. 3, 2000, pages 152-157, XP002960558 ISSN: 0027-769X cited in the application

## Description

### Field of the Invention

The present invention relates to the field of satiety enhancement for the treatment of a variety of diseases and/or disorders associated with overeating and/or excessive caloric intake of a subject. In particular it relates to shelf-stable liquid compositions that provide an enhanced satiety effect upon ingestion resulting from the fact that they gel under the influence of the acidic environment of the stomach. The use of these compositions as a satiety enhancing and/or satiating composition in a method of treating or preventing any of a variety of diseases and/or disorders associated with overeating and/or excessive caloric intake of a subject as well as methods of preparing these compositions are also provided.

### Background of the Invention

The incidence of obesity and the number of people considered overweight in countries where a so-called Western diet is adopted has drastically increased over the last decade. Since obesity and being overweight are generally known to be associated with a variety of diseases such as heart disease, type 2 diabetes, hypertension and arthereosclerosis, this increase is a major health concern for the medical world and for individuals alike.

Furthermore, being overweight is considered by the majority of the Western population as unattractive.

This has led to an increasing interest by consumers in their health and has created a demand for products that help to reduce or control daily caloric intake and/or control body weight and/or bodily appearance.

Several solutions have been proposed to help individuals to control their weight. Among these solutions is the use of medicinal products that e. g. suppress the activity of enzymes in the digestive system. However the use of medicinal products is often not preferred unless strictly required for medical purposes.

Another proposed solution is to prescribe the individuals a specific diet, for example, a diet with a restricted caloric intake per day. A problem with these diets is that often they do not provide a healthy nutritional balance and/or they are difficult to accommodate in modern lifestyles.

Meal replacer products have also been proposed as part of a healthy diet in order to control or reduce body weight. For example, US 5,688, 547 discloses a nutritional meal replacement composition comprising dietary fibre, protein and a cellulose gum and gel.

These meal replacer products are generally products that are intended to be consumed as a single-serving food product, such as a bar, drink etc to replace one or two meals per day. The meal replacer products are designed such that on the one hand they provide a restricted caloric intake, but on the other hand they provide a healthy balance of nutritional ingredients and are convenient to incorporate into an individual's daily diet.

However, a general problem with products intended to be used in a weight loss or weight maintenance plan, e. g. meal replacer products or low-calorie snacks, is that feelings of hunger may occur sooner than desired after consumption and/or the feeling of satiety obtained may not be as great as desired. Both of these considerations may render it difficult for the individual to adhere to the plan or it may make it and/or the products used therein less appealing to consumers.

Recognizing the demand for effective and convenient satiety- inducing food products, research has been carried out to try to address the problems associated with the above approaches to controlling or reducing body weight.

One approach to addressing the aforementioned problems has been to investigate the use of satiety agents in food products in order to increase the satiety effect obtained from consuming a food product comprising the satiety agents.

Alginates, and their derivatives, have been used in food compositions claiming to have an advantageous satiety effect.

WO 01/17377 discloses uronic acid-containing polysaccharides cross-linked to each other to form a sponge-like structure that dissolves poorly in water and gastro-intestinal fluids, and which are poorly reabsorbed, in order to provide a satiety effect.

WO 02/096223 discloses a method of blunting the post-prandial glycemic response in humans by feeding an induced viscosity fibre system. The system comprises a lightly hydrolysed starch, a soluble dietary fibre source and acid-soluble multi-valent cations. Digestive enzymes act upon the lightly hydrolysed starch to produce an increase in viscosity of the system.

US 5 283 076 and US 5 324 526 disclose beverage formulations that may be used as health foods. The beverages preferably comprise 5-20% wt of low molecular weight alginates. Use of these alginates in the prevention of obesity is proposed.

US 5,688, 547 discloses shakes, puddings or mousses comprising protein, cellulose gel and gum and dietary fibres including pectin, alginate, gum arabic and guar gum.

WO01/56404 discloses that 0.01 to5% wt of a low molecular weight polymannuronate derived from alginate may be used in a functional beverage.

US 2003/0013679 and WO02/096353 disclose a method of blunting the post-prandial glycemic response in humans by feeding an induced viscosity fibre system comprising a lightly hydrolysed starch and a soluble dietary fibre source in amounts of at least10% wt.

Wolf et al in the paper "Glycemic and insulinemic responses of non-diabetic healthy adult subjects to an experimental acid- induced viscosity complex incorporated into a glucose beverage", Nutrition, Volume 18, numbers 7/8,2002, disclose an acid induced viscosity complex comprising alginates.

WO 02/096219 relates to a method of blunting the postprandial glycemic response to a meal by feeding an acid controlled induced viscosity fiber system. The first component of the induced viscosity fiber system is anionic soluble fiber, such as alginate, pectin, low methoxy pectin, carrageenan, xanthan an gellan. The second component of the induced viscosity fiber system is water-insoluble, acid-soluble multivalent cations.

WO 03/053169 discloses a liquid satiety enhancing composition with a pH of more than 5, a viscosity below 50 mPa.s at a shear rate of 100 s⁻¹ and 20 °C, and a viscosity of at least 125% of the aforementioned viscosity at a pH 3 and a temperature of 37 °C; with a caloric density between 0 and 500 kcal per liter, the composition comprising between 0.01 and 5 wt.% of one or more polysaccharides selected from the group consisting of pectin, preferably LM pectin, and alginate, and between 0.01 and 3 wt.% calcium. WO 03/053169 does not disclose any liquid composition comprising mixtures of pectins and alginates.

WO05/020719 discloses an aqueous liquid or spoonable edible composition comprising at least 1% wt protein and from 0.1 to 5% wt of a biopolymer thickening agent which is not denatured or hydrolysed between pH 2 and 4, said composition having a gel strength at 37 °C and pH 2 of at least 10 KPa. According to WO 05/020719, the biopolymer thickening agent is preferably selected from alginates, pectins, carrageenans, amidated pectins, xanthans, gellans, furcellrans, karaya gum, rhamsan, welan, gum ghatti, gum Arabic and mixtures thereof. These compositions are claimed to have good satiety effects and to be beneficial for use in weight control plans.

WO2007044511 discloses a liquid composition having pH 3.5-5 comprising HM pectin and alginate used for Obesity and increase Satiety.

However, there is still a need in the art for edible compositions that provide a good satiety effect for consumers, especially those wishing to control their calorie intake and/or body weight.

In particular, there is a need for edible compositions which provide excellent satiety enhancing effects, which are of acceptable taste and texture for the consumer, which are convenient and/or economical to manufacture and which are stable during manufacture and storage.

It is an object of the invention to provide such liquid edible compositions as well as methods of using them as a satiety enhancing and/or satiating composition in a method of treating or preventing any of a variety of diseases and/or disorders associated with overeating and/or excessive caloric intake of a subject

### Summary of the invention

The present inventors have found that liquid compositions comprising specific combinations of pectins and alginates, constitute excellent satiety enhancing and/or satiating compositions that can be used to reduce the tendency of a subject to overeat and/or to reduce caloric intake of a subject. The present compositions have acceptable taste and texture characteristics, are convenient and/or economical to manufacture and, in particular, are stable during manufacture and storage

These compositions are particularly effective in enhancing feelings of satiety and/or inducing satiation upon ingestion, which, without wishing to be bound by theory, is hypothesized to result from the fact that these compositions gel in the strongly acidic environment that prevails in the stomach. More in particular, these compositions were found to form gel particles after ingestion that are large enough not to leave the stomach until grounded down by the shear forces in the stomach.

In addition, it is believed, that the particular strength of these gel structures effectively increases the ability thereof to withstand the grinding forces of the stomach, the maximum grinding force thereof having been found to be close to 0.65 N (Marciani et al., "Assessment of antral grinding of a model solid meal with echo-planar imaging, Am. J. Physiol. Gastrointest. Liver Physiol. 280, 844-849, 2001). It has also been found that these gel structures exhibit exceptionally little syneresis as compared to gel systems formed by the prior art satiety enhancing compositions.

The gel particles may therefore induce a sense of fullness over a particularly long period of time, thus aiding their efficacy in treating or preventing any of a variety of diseases and/or disorders associated with overeating and/or excessive caloric intake of a subject.

As will be explained in more detail hereafter, preferred embodiments of the present invention provide liquid compositions that are even more effective in enhancing feelings of satiety and/or inducing satiation, *inter alia* because of further increased gel strength and/or further reduction of synersis. Other preferred embodiments are also provided by the invention, wherein storage stability of the composition is improved, viscosity of the composition is particularly low and/or mouthfeel is improved.

Compositions comprising mixed alginate pectin gel systems have been described by Mancini et al. (Nahrung 44 (2000), vol. 3, 152-157). The teachings of Mancini et al. relate to the field of restructured food products. The gelled systems disclosed by Mancini et al. have been prepared by mixing a drum dried fruit puree with water, heating said mixture, adding a mixture of water and GDL (glucono-delta-lactone), a slow acidifier, and subsequently adding a mixture of alginate and water. By the addition of GDL the pH of the formulation is lowered to a value within the range of 3.54 to 3.85. After addition of the alginate a gel was reported to be immediately formed as a result of the low pH and/or of the heating of the fruit puree prior to addition of the alginate. Mancini et al. do not disclose any liquid mixture of pectins and alginates that remains stable upon storage and is capable of forming a gel in situ when the pH is lowered. Liquid mixtures of pea or carrot puree with alginates having a pH of 4.9 described by Mancini et al. did not form gel upon lowering the pH. Thus these mixtures are not suitable for use as a satiety inducing composition.

US 5,866,190 discloses a drink based on fruits and/or vegetables, comprising insoluble components, said composition comprising a combination of a pectin and of an alginate in which the ratio of the amount of methylated galacturonic acid (MGA) units of the pectin to the amount of guluronic acid (G) units of the alginate is between 0.30 and 0.70 as a stabiliser. The alginate has a G/M ratio of 0.5. This document does not relate to satiety inducing effects. Moreover the total amount of pectin and alginate is always below 0.2 wt% and the pH is between 2.5 and 4.0.

EP 1 338 210 concerns stabilisation of aqueous acidic protein suspensions comprising high methoxyl pectin and propylene glycol alginate, a synthetic alginate, in a combined weight of 10 to 70 % of the weight of the protein. The use of propylene glycol alginate allows for these compositions to have a pH well below 3, without a gel being formed.

EP 1 488 812 concerns phramaceutical formulations for targeted delivery of therapeutic agents to stomach and/or duodenal ulcers. These formulations utilize a liquid matrix undergoing phase transfer in vivo. The compositions of EP 1 488 812 may contain combinations of cross-linkable water-soluble polymers as well as insoluble salts releasing polyvalent cations under acidic conditions. EP 1 488 812 does thus not teach the use of acidic liquid compositions. Furthermore, EP 1 488 812, teaches to use LM pectin instead of HM pectin when a gel of high physical strength is required.

### Detailed Description of the Invention

The invention is best described by the claims 7 and 9. Thus, in a first aspect, the present invention relates to a shelf-stable liquid composition comprising a combination of:
i) 0.05-5.0 wt% of HM pectin;
ii) 0.1 - 5.0 wt% of alginate; and
iii) 50 - 99 wt% of water;
having a pH of greater than 3.6 and below 5.0, wherein the viscosity of the liquid composition is within the range of 0.003-1.0 Pa.s at a shear rate of 10 s⁻¹ and a temperature of 20 °C said composition having a force to fracture of 0.65-12 N at a temperature of 37°C and a pH < 3.5. said composition being consumed in a volume of 100 to 300 ml by person for enhancing feelings of satiety and/or inducing satiation upon ingestion

As mentioned herein before, the viscosity of the liquid composition is within the range of 0.003-1.0 Pa.s at a shear rate of 10 s⁻¹ and a temperature of 20°C. More preferably, under the aforementioned conditions, the viscosity is within the range of 0.005-0.8 Pa.s, still more preferably 0.008-0.7 Pa.s, most preferably the viscosity is within the range of 0.01-0.5 Pa.s. Unless specifically stated otherwise, any reference in this document to viscosity values refers to a The invention is best described by the claims 7 and 9, physical characteristic/parameter of the composition that is defined as the ratio of shear stress to shear rate, expressed as dynes/cm², poise, centipoise (cps), which is one hundredth of a poise or Pascal second (Pa.s), which equals one tenth of a poise. A poise is a unit of coefficient of viscosity, defined as the tangential force per unit area required to maintain one unit difference in velocity between two parallel planes separated by one centimetre of fluid. According to the present invention, viscosity determinations are carried out at a shear rate of 10 s⁻¹, for example, on a AR 1000 rheometer from TA instruments.

The term "shelf-stable" in accordance with the present invention means that the physical state of liquid composition remains essentially the same upon storage, or, more in particular, that the viscosity of the composition will remain within the above mentioned ranges, when the liquid composition is refrigerator-stored or shelf-stored, i.e. at any temperature of between 4 °C and room temperature, typically for a period of at least 6 months, more preferably for a period of at least 9 months, most preferably for a period of at least 12 months. In the context of the present invention, the term shelf-stable is not intended to refer to any type of spoilage such as microbial spoilage, off-flavour-development or the like.

As mentioned herein before, the present liquid compositions are furthermore characterized by the fact that solidification/gelation will be observed upon lowering the pH to values corresponding to those typically found in the stomach. The gels formed under said conditions typically have a force of fracture within the range of 0.5 - 12 N, more preferably within the range of 0.7 - 10 N, most preferably within the range of 0.75 - 8.5 N. Unless specifically stated otherwise, any reference in this document to "the force of fracture" is meant to refer to a physical characteristic/parameter of the composition that is determined by a compression test of gelled polymer cylinders. According to this method, polymer gel cylinders are prepared by pouring the polymer solution containing glucono-delta-lactone as a slow acidifier into teflon moulds (H=12mm and d=12mm). The moulds containing the polymer solution are then equilibrated at 37°C for 3 hours in order to reduce the pH to below 3.5. The gels are subsequently aligned in the center of stainless steel compression plates which are lubricated with mineral oil to reduce friction and the force required to fracture the gels can be measured using a Texture Analyser (XT Plus) and a head speed of 2.5 mm/sec.

The term "comprising" whenever used in this document is intended to indicate the presence of stated features, integers, steps, components, but not to preclude the presence or addition of one or more other features, integers, steps, components or groups thereof.

As used herein, the term "satiety" is meant to refer to an essentially homeostatic state wherein an individual feels that their cravings are satisfied or minimized. Many physiological factors are believed to bear on an individual's satiety. For instance, gustation, or taste, olfaction, or smell, as well as a feeling of fullness of the stomach may all contribute to whether an individual feels "satiated." More in particular, "satiety" is the sate in which further eating is inhibited and determines the time between meals and the amount of food consumed at the next meal (Burton-Freman, 2000). Whenever reference is made in this document to an "enhanced feeling of satiety" or the like, this has the meaning of the feeling of satiety being more pronounced and/or more prolonged compared to a control situation. It will be clear to a person skilled in the art what is meant with a 'control situation' in the context of the present invention, for example a situation wherein a subject is given a liquid composition that does not form a gel *in situ* instead of an equal volume of a liquid composition according to the present invention.

The term "satiation", as used herein refers to the state which terminates eating within a meal, typically occurring/observed within 60 min. after the start of consuming the meal. Thus, whenever reference is made in this document to "inducing satiation" or the like, this has the meaning of arousing the tendency of a subject to stop consuming food during a meal.

As used herein the term "syneresis" refers to the spontaneous exudation or segregation of liquid from the gel, accompanied by the shrinkage of the said gel. Syneresis can be expressed as the amount (in grams) of liquid segregated from a specified amount of the gel over a specified period of time. The same characteristic can also be expressed as the percentage of volume decrease of the gel over a specified period of time.

Pectins are a common type of carbohydrate gelling agent, generally obtained from dilute acid extracts of citrus or apple pulp. They are an important constituent of the cell walls and soft tissue of vegetables and fruits, where they contribute to the mechanical properties of the cell wall and influence cell adhesion. Pectins are for example found in root crops such as carrots and beetroot, as well as in tubers, such as potatoes and are commercially extracted from citrus peels, apple pomace and sugar beet pulp. Pectin is composed of long, regular sequences of 1,4-linked-D-galacturonate residues which in nature may be partially methyl-esterified. A typical pectin molecule comprises 200 to 1000 galacturonic acid units connected in a linear chain having alternating rhamnose units inserted into the main uronide chain. The ester content varies with the source of the raw material and may also be varied during extraction. Pectins are divided into two main categories: high methoxylated pectins (hereafter referred to as HM pectin), which are characterized by a degree of methoxylation above 50%, more particularly between 50% and 80%, and low methoxylated pectin (hereafter referred to as LM pectin) having a degree of methoxylation below 50%, more particularly between 30% and 50%. As used herein,"degree of methoxylation" is intended to mean the extent to which free carboxylic acid groups contained in the polygalacturonic acid chain are present as the methyl ester.

Both HM and LM pectins form gels. However, these gels form via totally different mechanisms (Voragen et al, In Food polysaccharides and their applications. pp 287-339. Marcel Dekker, Inc. New York, 1995). LM pectin forms a gel in the presence of calcium, thus, it is "calcium-reactive." The calcium-LM pectin gel network is built by formation of what is commonly referred to as an "egg-box" junction zone in which Ca²⁺ causes the cross-linking of two stretches of polygalacturonic acid chains. Calcium-LM pectin gel formation is influenced by several factors, including DM, ionic strength, pH, and molecular weight. Furthermore, the calcium-LM pectin gelation is more efficient at a neutral pH of about 7.0 than at an acidic pH of about 3.5. Lastly, the addition of monovalent counter ion (NaCl) enhances the gelation, i.e., less calcium is required for gel formation. HM pectin forms a gel in the presence of high concentrations of co-solutes (sucrose) at low pH. HM pectins are generally not reactive with calcium ions and therefore cannot form a calcium gel. However, certain HM pectins have been reported to be calcium sensitive and capable of calcium gel formation. In addition, HM pectins can be made calcium-reactive by a block wise de-esterification process while still having a DM of >50%. See, Christensen et al. U.S. Pat. no. 6,083,540.

Pectins are typically utilized in the food industry and classified by the FDA as "GRAS" (Generally Regarded As Safe). They have also long been used as colloidal and anti-diarrhea agents. Recently, pectins have been utilized in the areas of medical device and drug delivery (Thakur et al., Critical Reviews in Food Science & Nutrition 37, 47-73, 1997). In the case of drug delivery, pectin has found its presence in many experimental formulations for oral drug delivery to the colon because pectin is readily degraded by bacteria present in this region of the intestines.

According to the present invention, the shelf-stable liquid composition comprises 0.05-5.0 wt% HM pectins, which are preferably characterized by a degree of methoxylation of above 50%, preferably within the range of 50-90 %, still more preferably within the range of 55-85 % most preferably within the range of 60-80 %.

It has been found by the present inventors that the molecular weight of the pectin does not affect the gel strength of the gel formed upon ingestion, but does affect the viscosity of the liquid composition itself. More in particular, it was found that low molecular weight HM pectins form similarly strong interactions with alginate as do high molecular weight HM pectins. Thus, the addition of low molecular weight HM pectin will reduce the viscosity of the product and improve the mouth feel thereof, without a concomitant decrease in strength of the gel particles formed in the stomach as compared with the use of high molecular weight HM pectins. Therefore, according to a preferred embodiment, shelf-stable liquid composition are provided, wherein the average molecular weight of the HM pectin is within the range of 50-500 kDa, more preferably within the range of 75-250 kDa, still more preferably 90-200 kDa.

HM pectins are used according to the present invention since, in combination with alginates, they are capable of forming a sufficiently rigid matrix at a pH found in the stomach of a normal human, typically a pH of below 3.5. Pectins cannot be included unrestrictedly in the present liquid composition, since at high concentrations the composition will acquire an unacceptable high viscosity. On the other hand, a significant concentration of pectin is required to provide a satiety inducing effect and/or satiation. Hence the liquid composition used in the present method preferably contains 0.08 - 2.5 wt. %, preferably 0.1 - 1.0 wt. %, more preferably 0.2 - 0.8 wt. % of HM pectin based on the total weight of the liquid composition.

Alginates are a heterogeneous group of linear binary copolymers of 1-4 linked β-D-mannuronic acid (M) and its C-5 epimer α-L-guluronic acid (G), with a wide range of average molecular weights (100-100,000 residues). The monomers are arranged in a blockwise pattern along the polymeric chain where homopolymeric regions (M blocks and G blocks) are interspaced with sequences containing both monomers (MG blocks). Only the G-block is responsible for calcium gelation. The total G content varies widely dependent on the sources. The proportion and sequential arrangement of the uronic acids in alginate depend upon the species of algae and the kind of algal tissue from which the material is prepared. Various properties of different types of alginates are based upon the guluronic acid makeup of the particular alginate. For example, viscosity depends mainly upon the molecular size, whereas the affinity for divalent ions essential for the gel-forming properties are related to the guluronic acid content. Specifically, two consecutive di-axially linked G residues provide binding sites for calcium ions and long sequences of such sites form crosslinks with similar sequences in other alginate molecules, giving rise to gel networks.

Commercial alginates are produced mainly from Laminaria hyperborea, Macrocystis pyrifera, Laminaria digitata, Ascophyllum nodosum, Laminaria japonica, Eclonia maxima, Lesonia negrescens and Saragassum sp.

The material used in the present invention is alginate comprised substantially of α-L-guluronic acid (G) which may be referred to herein as guluronic acid, and mannuronic acid (β-D-mannuronic acid) (M). For use in this invention, alginate can be prepared according to methods well known in the art. For example, alginate can be commercialy obtained from numerous outlets including Sigma (St. Louis, Mo.) and Protan A/S (Drammen, Norway). Poly G alginate may be obtained from Protan (Norway or Seattle), or may be obtained by isolation of the material from natural sources or by chemical conversion by methods reported in the literature.

In accordance with a particularly preferred embodiment of the invention Shelf stable liquid compositions as defined herein before are provided, wherein less than 80 %, preferably less than 60 %, more preferably less than 40 % and most preferably less than 20 % of the total amount of guluronate and mannuronate units is esterified. It is particularly preferred that the alginate does not contain any esterified M or G units.

According to another preferred embodiment of the invention, shelf stable liquid compositions as defined herein before are provided, wherein the alginate is not a propylene glycol alginate.

A preferred embodiment provides a shelf-stable liquid composition according to the present invention comprising alginate having a guluronate content of at least 50%, preferably at least 60 %, more preferably at least 65 %, still more preferably at least 70 %, and most preferably at least 85 %, which is particularly effective in enhancing feelings of satiety upon ingestion. The amount of mannuronic acid residues is preferably less than 50 %, more preferably less than 40 %, still more preferably less than 30 %, most preferably less than 15 %. Furthermore, particularly suitable satiety enhancing compositions comprise alginates having a G block-content of at least 25%, more preferably at least 50%, most preferably at least 60%.

Yet in another preferred embodiment, shelf-stable liquid compositions are provided, wherein the average molecular weight of the alginate ranges from 100-400 kDa, more preferably from 200-350 kDa, still more preferably from 250-325 kDa. It was found that these compositions are particularly effective in enhancing feelings of satiety upon ingestion, which, without wishing to be bound by theory, is hypothesized to result from the fact that these compositions upon lowering the pH form gels that are particularly strong and/or exhibit particularly little syneresis.

According to the present invention alginate cannot be included unrestrictedly in the present liquid composition, since at high concentrations the composition will acquire an unacceptable high viscosity. On the other hand, a significant concentration of alginate is required to provide a satiety inducing effect and/or satiation. Hence the liquid composition used in the present method preferably contains be alginate in an amount within the range of 0.1 - 2.5 wt%, more preferably within the range of 0.2-1.5 wt%, still more preferably within the range of 0.4 - 1.0 wt%, based on the total weight of the composition.

According to a particularly preferred embodiment of the invention, the total amount of the combination of the pectin and the alginate ranges from, 0.2 - 5.0 wt%, more preferably from 0.3 - 2.0 wt%, most preferably from 0.5 - 1.5 wt%, based on the total weight of the composition.

The shelf-stable liquid composition of the present invention furthermore comprises water. As will be explained in more detail hereafter, said composition may comprise a variety of additional ingredients, some of which may influence the viscosity of the composition. The total amount of water that is to be used in accordance with the present invention will typically be such as to provide the required viscosity and volume to the product, and may therefore vary widely typically from 25-99 wt%, more preferably from 50-99 wt%, still more preferably from 60-99 wt%, most preferably from 70-99 wt%.

According to a preferred embodiment of the invention the present shelf-stable liquid compositions have a pH of more than 3.8, preferably more than 3.85, more preferably more than 3.9, most preferably more than 4.0. Most preferably the present liquid compositions have a pH that does not exceed 5.0, more preferably it does not exceed 4.8, most preferably it does not exceed 4.6.

The mechanical properties of the pectin alginate mixed gels, which will form upon further acidification, typically to pH values corresponding to those in the stomach, depend on the pectin-to-alginate ratio, the mannuronic acid and guluronic acid ratio of the alginate and the DM of the pectin. The nature of the interactions between pectin and alginates in mixed gels is not well-known. Without wishing to be bound by theory said interactions appear to involve a heterogeneous association between specific chain sequences of two polymers: alginate poly-L-guluronate blocks and pectin poly-D-galacturonate sequences of low charge density. The interaction between pectin and alginate is enhanced as the proportion of these sequences is increased. Although the conformation of individual chains is the same as in homotypic, calcium-mediated junctions, the geometry of the interaction is quite different, and instead of leaving cavities capable of accommodating metal ions, the near mirror-image chains form a close packed, nested structure. This results in favourable non-covalent interactions between methylester groups of pectin and the H-1 and H-2 of the polyguluronate. For LM pectin a much lower pH is required to form a gel with high-G alginates. The melting point of these gels increases with decreasing pH.

According to a particularly preferred embodiment of the present invention the shelf stable liquid composition comprises the combination HM pectin and alginate as mentioned herein before, the ratio of pectin to alginate being within the range of 0.1-10, more preferably, within the range of 0.1-5, still more preferably within the range of 0.2-1.0, most preferably within the range of 0.3-0.8. It was found that these compositions are particularly effective in enhancing/prolonging feelings of satiety and/or inducing satiation upon ingestion, which is hypothesized to result from the fact that these compositions upon lowering the pH form gels, typically gel particles as mentioned before, that are particularly strong and/or exhibit particularly little syneresis.

Without wishing to be bound by theory, the present inventors believe that HM-pectin/high-G-alginate interactions are negatively affected by the presence of divalent and trivalent ions, resulting in a decreased gel strength of the synergistic mixed acid gel of alginate and HM pectin. It is known that calcium alginate gels formed at neutral pH values shrink if the calcium concentration is higher than a certain ratio of calcium to alginate. For example, it is known that a 1% alginate gel shrinks at calcium concentrations exceeding 0.05 mol/l (Shchipunov et al., 2002). The inventors have found that gels formed of the present liquid compositions under acidic conditions will significantly shrink at calcium concentrations as low as 0.004 mol/1. In accordance with the present invention, it is therefore preferred that the concentration of soluble di and trivalent metal ions is below 0.01 mol/liter, preferably below 0.006 mol/liter, most preferably below 0.004 mol/liter.

Interestingly it was also found that although the HM pectin / alginate mixture requires a pH below 3.5 to form a gel, the presence of potassium increased the onset of gelation. Thus, it is important to control the potassium levels of the liquid compositions as to have a stable product at a pH of between 3.6 and 5.0. However, the presence of the right amount of potassium will decrease the time for gelation onset in - vivo. According to a preferred embodiment, the concentration of potassium in the composition is below 0.2 mol/liter, more preferably below 0.1 mol/liter.

The presence of sugars like sucrose and fructose does not seem to have significant impact on the pH of gelation of the present composition but has been found to increase the force required to fracture the pectine alginate mixed acid gels. Therefore, according to another preferred embodiment, the present liquid composition comprises carbohydrates, preferably disaccharides, typically in amounts of between 0.5-20 wt%, more preferably 1-10 wit%.

As mentioned herein before, part or all of the HM pectin to be incorporated in the present shelf-stable liquid composition can be comprised in a fruit and/or vegetable composition and added as such to said liquid compositions. According to this embodiment the present shelf-stable liquid compositions typically comprise an amount of fruit and/or vegetable constituents that is sufficient to provide the amounts of HM pectins as specified here above. Typically, depending on the type of the fruit and/or vegetable solids used, said amount may, based on the total weight of the liquid compositions, range from 0.1 to 30 wt%, preferably from 0.5 to 20 w%, more preferably from 1 to 15 wt% still more preferably from 2 to 10 wt% and most preferably from 3 to 7 wt% of fruit and/or vegetable constituents other than water, i.e. fruit and/or vegetable solids. The term "fruit solids" as used herein refers to the dry matter contained in any fruit material that is incorporated in the edible product. It will be clear to the skilled person that the aforementioned percentages refer to the equivalent amount of dry fruit solids that is incorporated. According to a preferred embodiment, the fruit solids originate from one or more of the following fruit sources: citrus fruit (e.g orange, tangarine, lemon or grapefruit); tropical fruit (e.g. banana, peach, mango, apricot or passion fruit); red fruit (e.g. strawberry, cherry, raspberry or blackberry), or any combination thereof. Vegetable solids in accordance with the present invention can originate from one or more of the following sources: tomatoes, carrots, peas, celery and beetroot, sugar snaps, haricot verts, corn and beans. According to a further preferred embodiment, fruits and/or vegetables are used with a relatively high pectin content, preferably a relatively high content of HM pectin, such as citrus fruits and/or tomatoes.

Advantageously, the fruit and/or vegetable solids employed in the present process comprise at least 1 %, more preferably at least 5 % and most preferably at least 10 % of pectin, preferably HM pectin, calculated on the basis of the dry weight thereof. The fruit and/or vegetable solids can be incorporated in the present liquid compositions in any suitable form, for example, as intact fruit and/or vegetable, as a puree, a juice, as a comminuted product, as chunks or as a blend of these products. Preferably the fruit and/or vegetable is added in fluid form e.g. as a juice or a puree having a viscosity expressed in Bostwick consistometer values of between 5 and 20 cm. at 20°C.

Depending, amongst others, on the intended use as will be explained hereafter, the present compositions may optionally contain a variety of other functional ingredients, such as those mentioned here below. The levels of these ingredients may vary in a broad range for example for each of these ingredients up to 15 wt%.

According to one embodiment, the present liquid compositions comprise agents that are known to delay gastric emptying and increase intestinal transit time. Suitable examples thereof include soluble fibres, such as guar gum, gum Arabic, karaya, tragacnth and psyllium.

According to another embodiment, agents that effectively lower LDL-cholesterol levels and/or blood glucose levels may be incorporated in the present liquid compositions. Such agents can be selected from the group of soluble fibres with water holding capacity, such as beta-glucan, oat bran, psyllium, glucomannan, konjac mannan, pectin, psyllium and the like.

According to yet another embodiment of the present invention, the present liquid compositions comprise insoluble fibres such as cellulose and hemicellulose. These agents have a stool-bulking effect and therefore can speed up the rate of passage through the colon and prevent constipation.

Inulin and fructo-oligosaccharides (FOS) can selectively stimulate bifido bacteria in the human colon and improve the integrity of the intestinal barrier. In addition, inulin and FOS can increase the absorption of divalent cations such as calcium in the colon, which can be hindered by alginates. Thus, in another embodiment of the invention, the liquid compositions comprise inulin and/or fructo-oligosaccharides.

The liquid compositions of the invention may comprise optionally encapsulated satiety agents which are predominantly released in the intestines. Suitable satiety agents include lipids, especially mono, di or tri-glycerides, their free fatty acids, their edible salts, their non-glyceryl esters, hydrolysable in the presence of gastro-intestinal enzymes, and mixtures thereof. These satiety agents may be encapsulated in any suitable cross-linked encapsulating agent whereby they are predominantly released in the intestines.

The liquid compositions of the invention, according to a first embodiment, may comprise peptides, which could positively influence satiety enhancement. A suitable example thereof includes a whey protein hydrolysate which is capable of inducing the cellular release of glucagon-like-peptides and cholecystokinins.

The liquid compositions of the invention may optionally comprise, in suitable amounts, one or more agents which may beneficially influence (post- prandial) energy metabolism and substrate utilisation, for example caffeine and/or, flavonoids (including tea catechins, capsaicinoids and carnitine).

To increase the palatability of the present liquid compositions artificial sweetener and/or natural sugars, salts and/or flavouring may be incorporated. Artificial sweeteners may comprise a low-caloric artificial sweetener, which preferably is selected from indigestible oligosaccharides, polyols, aspartame, sucralose, acesulfame potassium, alitame, cyclamate, saccharin, tagatose and mixtures thereof. Flavourings may be any of the commercial flavours natural and/or artificial flavours, such as fruit flavours, e.g. orange, apple or lemon but also flavours of cocoa, pure vanilla, vanillin, ethyl vanillin, chocolate, malt, mint, yogurt powder, extracts, spices, such as cinnamon, nutmeg and ginger, mixtures thereof, and the like. It will be appreciated that many flavour variations may be obtained by combinations of the basic flavours. Flavourings which mask off-tastes from vitamins and/or minerals and other ingredients may be included in the edible compositions.

The liquid compositions may comprise one or more conventional colouring agents, preservatives, anti-oxidants, thinners, emulsifiers etc., which will be applied in conventional amounts known to the skilled person.

The compositions may comprise one or more cholesterol lowering agents in conventional amounts. Any suitable, known, cholesterol lowering agent may be used, for example isoflavones, phytosterols, soy bean extracts, fish oil extracts, tea leaf extracts.

The present liquid compositions may suitably comprise 0.05-5 wt%, preferably 0.1-2 wt% of vitamins, preferably selected from Vitamin A Palmitate, Thiamine Mononitrate (VitaminB1), Riboflavin (Vitamin B2), Niacinamide (Vitamin B3), d-Calcium Pantothenate (VitaminB5), Vitamin B6, VitaminB11, Cyanocobalamin (Vitamin B12), biotin, Ascorbic acid (Vitamin C), Vitamin D, Tocopheryl Acetate (Vitamin E), Biotin (Vitamin H), and Vitamin K. The vitamins and/or may be added by the use of vitamin premixes, and mixtures thereof or alternatively they may be added individually.

According to a preferred embodiment of the invention, the present liquid compositions do not contain any pharmacologically active substances selected from the goup of antibiotics, antibacterial agents and agents for treating and/or preventing gastritis, stomach ulcers and/or duodenal ulcers.

According to another embodiment, the present liquid compositions may suitably comprise carbohydrates as an energy source, e.g. food grade starches in an amount ranging from 0.5-4 wt%. It will be understood by the skilled person that the intake of food being reduced substantially after ingestion of the present liquid composition, may in some cases be accompanied by acute symptoms of insufficient energy intake, e.g. faintness or feelings of weakness, which may be balanced by the addition of an energy source, without departing from the scope of the invention.

Typically, the shelf-stable liquid compositions according to the present invention preferably have a caloric content within the range of from 0 kilocalories (kcals) to 500 kcals, more preferably 1 kcals to 400 kcals per 100 ml, still more preferably 2 kcals to 200 kcals per 100 ml. Most preferably the present liquid compositions have a caloric content of between 5 -100 kcals per 100 ml. According to this embodiment of the present invention the afore defined liquid compositions can suitably be presented in the form of a fruit drink, a smoothy, a yoghurt drink, a dairy drink, a soup, a tea based drink or the like. A satiety enhancing and/or satiating energy drink, i.e. a composition in accordance with the invention, including carbohydrates as an energy source, is however also encompassed.

The caloric content of a composition can be suitably calculated by multiplying the caloric density of an ingredient per gram with the weight of the ingredient included in the composition. The cumulative value gives the caloric content of the composition.

In another preferred embodiment, shelf stable liquid composition as defined herein before are provide by the invention, excluding aqueous acidic protein suspensions comprising high methoxyl pectin and propylene glycol alginate in a combined weight of 10 to 70 % of the weight of the protein. As mentioned herein before, the shelf-stable liquid compositions according to the present invention typically form gels or gel particles at a pH of below 3.5 and at a temperature of approximately 37 °C, said gels being characterised by a force to fracture within the range of 0.65-10 N. Because these conditions correspond to those in the stomach of a mammal, in particular monogastric mammals, such as humans, the present shelf-stable liquid compositions can be used as satiety inducing or enhancing compositions in treatment programs involving reducing and/or controlling the caloric intake of a subject, as will be explained hereafter. When ingested the drink reaches the stomach and starts to gel due to the acid environment of the stomach, which typically involves a pH value of below 3.5. Due to the formation of this viscous/solidified matrix in the stomach, this formulation is cleared/emptied from the stomach much more slowly than a liquid that remains liquid in the stomach leading to an enhanced and/or prolonged feeling of satiety and/or to more pronounced satiation, which in turn will lead to the intake of food being reduced subsequently to ingestion of the present liquid composition.

Furthermore, without wishing to be bound by theory it is believed that the formation of said viscous/solidified matrix may in addition increase the release of certain cholecystokinins that are known to aid to feelings of satiety, although the exact mechanism involved therein may not have been entirely clarified yet. Consuming a composition according to the invention is thus intended to enhance the feeling of satiety for the consumer and/or to induce satiation, which may typically lead to extension of the time interval between subsequently taken meals and/or a reduction in the amount of calories consumed in the following meal. Thus, as a results of the satiety inducing effects, the shelf-stable liquid compositions according to the present invention can advantageously be used in a method of reducing appetite and or reducing the tendency to overeat in a human subject, such that the caloric intake of said subject will typically be reduced when the liquid composition is consumed shortly before or during a meal

Hence, the present shelf-stable liquid composition is advantageously used for treating and/or preventing any disease or disorder that is associated with overeating and/or excessive caloric intake. Suitable examples of such diseases and/or disorders include obesity, hypercholesterolemia, hyperlipidemia, atherosclerosis, hypertension, diabetes mellitus type 2, hyperglycaemia, insulin resistance, and metabolic syndrome. As mentioned herein before, in order to treat or prevent such diseases or disorders the present composition can be used to reduce feelings of hunger, aid in the adherence to a dietary plan, induce or enhance feelings of satiety and/or to reduce food-intake.

Alternatively the present liquid compositions may be used for any other method of treating a subject wherein the reduction of caloric intake and/or the reduction of appetite is beneficial to said subject for other (non-medical) reasons. A suitable example thereof includes losing weight for cosmetic reasons, i.e. for improving self-image.

Therefore, another aspect of the present invention relates to the Use of the shelf-stable liquid composition as defined in any one of the preceding claims for the manufacture of a liquid preparation for use in a method of prophylactic or curative treatment of obesity, being overweight, hypercholesterolemia, hyperlipidemia, hyperglycaemia, insulin resistance, diabetes mellitus type 2 or metabolic syndrome in a mammal and to reduce feelings of hunger, aid in the control of satiety and reduce the food intake.

In a preferred embodiment of the invention the aforementioned method comprises oral administration of at least one serving of the liquid preparation at least once a day, preferably within 60 minutes, more preferably within 30 minutes, most preferably within 15 minutes prior to a meal. In alternative embodiment of the present invention, the method comprises oral administration of at least one serving of the liquid preparation during a meal. the liquid preparation is administered in an amount of 100-300 ml per serving.

It will be clear to the skilled person however that the amount of the present liquid composition to be administered as well as the regime of administration to the subject in order to achieve the desired efficacy will vary, depending on the circumstances such as the disease or disorder to be treated, the desired effect to be achieved, and the condition of the subjects to be treated. It is however within the skill of a trained professional to determine the exact amounts required.

Compositions according to the present invention are typically prepared by any suitable conventional technique. Such techniques are well known to those skilled in the art and do not need to be described further here but may include mixing, blending, homogeniping, high-pressure homogenising, emulsifying, dispersing, or extruding. The composition may be subject to a heat treatment step, for example pasteurisation or U. H. T. treatment. A particularly preferred aspect of the invention relates to a method of producing a shelf-stable liquid composition as defined herein before, comprising blending water, alginate and HM pectin in the amounts and with the specifications described herein before; optionally, any of the aforementioned additional ingredients; and, if required to obtain a liquid composition in accordance with the invention, a pH adjusting agent. According to a particularly preferred embodiment said method comprises blending water, alginate and an amount of fruit solids in accordance with what has been specified herein before.

The present invention will be further illustrated hereafter by means of examples, which are in no way intended to limit the scope of the invention.

### Examples

### Example 1: Satiety enhancing and/or satiating liquid pectin/alginate liquid composition:

A liquid composition was prepared in accordance with the present invention by mixing a fruit/vegetable base, comprising water and concentrated fruit and vegetable juices, and a polymer solution. Said polymer solution contained alginate (Manugel DMB from ISP), having a mannuronate:guluronate content of 28:72, as determined by NMR in-house. The Mw of this alginate is 283.000 g/mol, as determined using HPSEC-MALLS. The polymer blend furthermore contained HM pectin (JM 150 from CP Kelco) having a methyl ester content of 70% its Mw is around 100 000 - 180 000 g/mol. The liquid composition is flavoured using an orange flavouring (Citronova 533 Orange). The exact formulation of the liquid composition is shown below in table 1.

**Table 1: formulation of a liquid composition of the invention**

| **Ingredient** | **% Formula** | **g per serving** |
|---|---|---|
| *Fruit + Veg Base:* | | |
| Water | 40,59 | 40, 99 |
| Orange Juice Conc (SVZ) | 3,40 | 3,43 |
| Carrot Juice Conc (SVZ) | 6,01 | 6,07 |
| *Polymer Solution:* | | |
| Water (Demineralised) | 44,82 | 45,27 |
| Pectin | 0,40 | 0,40 |
| Alginate | 0, 60 | 0, 61 |
| Sugar | 4,17 | 4,21 |
| *Flavouring:* | | |
| Orange Flavouring | 0,015 | 0,015 |
| **TOTAL** | 100, 00 | 101,00 |

The following characteristics of this liquid composition per serving have been determined:

| | |
|---|---|
| **Calories:** | 20.06 kcal |
| **Viscosity:** | 0.2 Pas at a shear rate of 10 s⁻¹ |
| **pH:** | 4.25 |
| **Gel strength (force to fracture):** | 2.5 N |

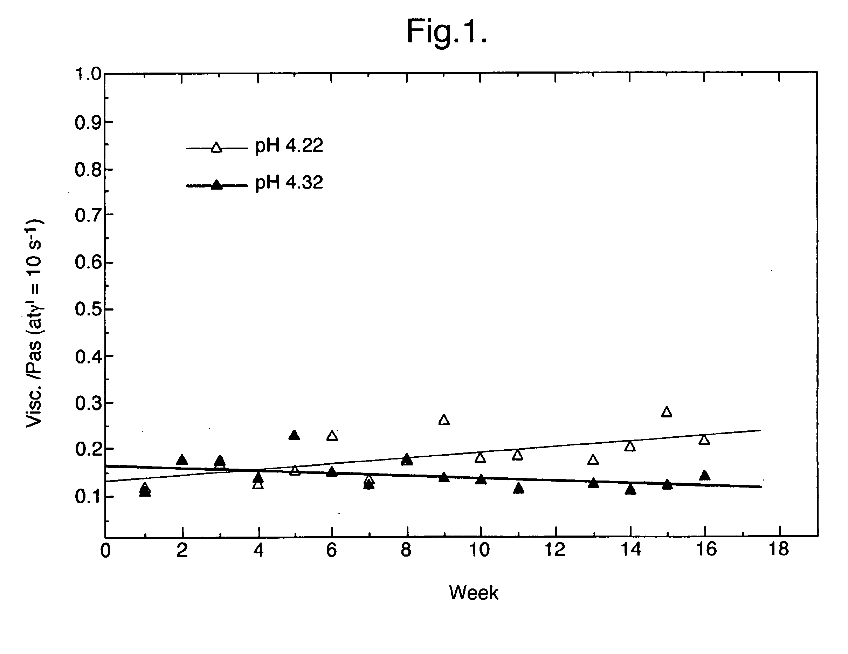

Figure 1 shows the viscosity (Pas., shear rate = 10 s⁻¹) of two samples having the above formulation over time during storage. Sample 1 was slightly more acidic than sample 2 (pH 4.22 vs. pH 4.32).

### Example 2: Efficacy of pectin/alginate liquid composition in reducing food intake:

A Randomised, double-blind, controlled, crossover study involving 24 subjects is performed. The subjects are Healthy male and female volunteers, age 18-60, BMI of ≥ 21.0 and < 32.0 kg/m². Individuals with any chronic diseases or malfunctioning of the stomach, pregnant or lactating females, individuals taking medications/supplements which may affect appetite and food intake or acid secretion in the stomach (antacids), individuals who demonstrate high eating restraint are excluded from entering the study.

Subjects receive in total 4 treatments. During each treatment day, subjects will be instructed to consume a conventional meal replacement bar for breakfast (appr. 08.00 hrs). Fifteen minutes before lunch (lunch at apr. 12.00 hrs), subjects will receive 200 ml of a satiety enhancing liquid composition according to the invention containing carrot/orange juice concentrate, water, sugars, HM-pectin and alginate (total polymer content is 1 % w/w, in the ratio of 40:60), or of a control liquid composition containing carrot/orange juice concentrate, water, sugars and thickeners to match the viscosity of the treatment composition. Lunch consists of either a conventional meal replacement beverage or an ad libitum meal (meal 1). Only when subjects received a conventional meal replacement beverage for lunch instead of an ad libitum meal, they will receive an ad libitum meal (meal 2) later in the afternoon (at appr. 14.30 hrs). Water consumption will be allowed throughout the day (with the exception of 30 minutes before liquid composition and ad libitum meal intake), but will be kept the same for each treatment day. The liquid compositions should be consumed within 3 minutes. Time points are indicative. The 24 test subjects are divided into 4 groups which are treated according to the following schemes:
Treatment 1: Consumption of the satiety enhancing liquid composition described in example 1, followed by an ad libitum lunch (meal 1). No ad libitum meal in the afternoon.
Treatment 2: Consumption of the control liquid composition in the morning followed by an ad libitum lunch (meal 1). No ad libitum meal in the afternoon.
Treatment 3: Consumption of the satiety enhancing liquid composition described in example 1 in the morning followed by a conventional meal replacement lunch. Subjects receive an ad libitum meal (meal 2) later in the afternoon.
Treatment 4: Consumption of the control liquid composition in the morning followed by a conventional meal replacement lunch. Subjects receive an ad libitum meal (meal 2) later in the afternoon.

Efficacy is assessed by measurement of voluntary energy intake during ad libitum meals and by subjects' self-assessments of feelings of hunger/satiety, fatigue and GI complaints (i.e. just before intake of liquid composition, just before lunch, after lunch (12.30 hrs), and every 30 min following lunch till 15.00 hrs.). Fatigue and GI complaints are measured just before snack consumption, at 13.30 hrs. and 15.00 hrs.

The voluntary energy intake during ad libitum meals is reduced significantly in the treatment groups, which corresponds to self-assessed feelings of hunger/satiety. No significant differences in fatigue and GI complaints are observed between the different treatment groups. Such results would imply that the treatment according to the invention is effective and without signs of unacceptable side-effects.

### Example 3: Gelation of pectin/alginate liquid composition in the stomach.

In two individual experiments a healthy volunteer ingested, on an empty stomach 100 ml or 250 ml, respectively, of a liquid composition having the fromulation shown in table 1.

The process of gel (lump) formation in the stomach was followed using an MRI apparatus. Images of the abdominal region were taken (a) before, (b) immediately after, (c) 15 minutes after and (d) 20 minutes after ingestion of the liquid composition of the invention.

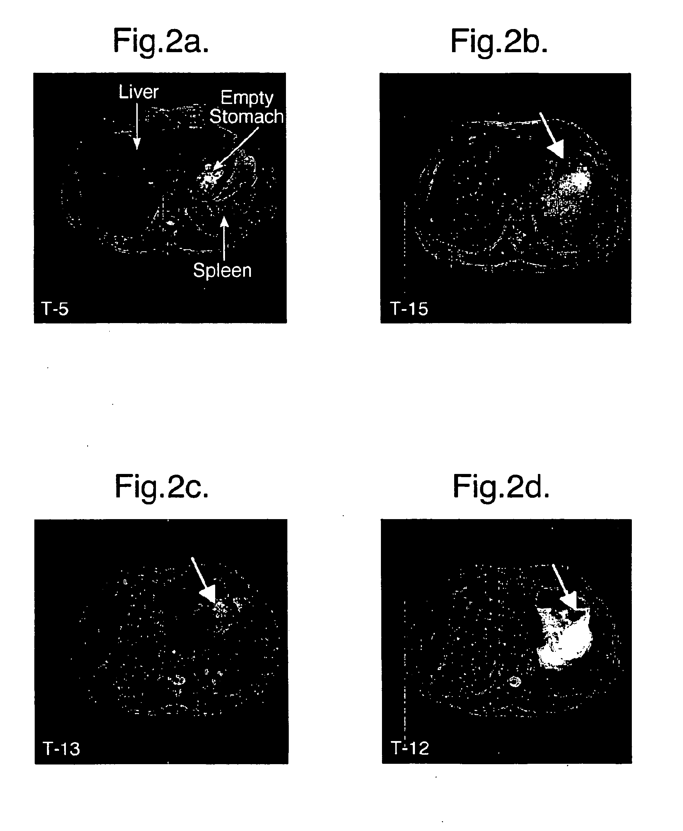

Figure 2 shows the respective MRI images of the stomach of the volunteer that ingested 100 ml of the composition.

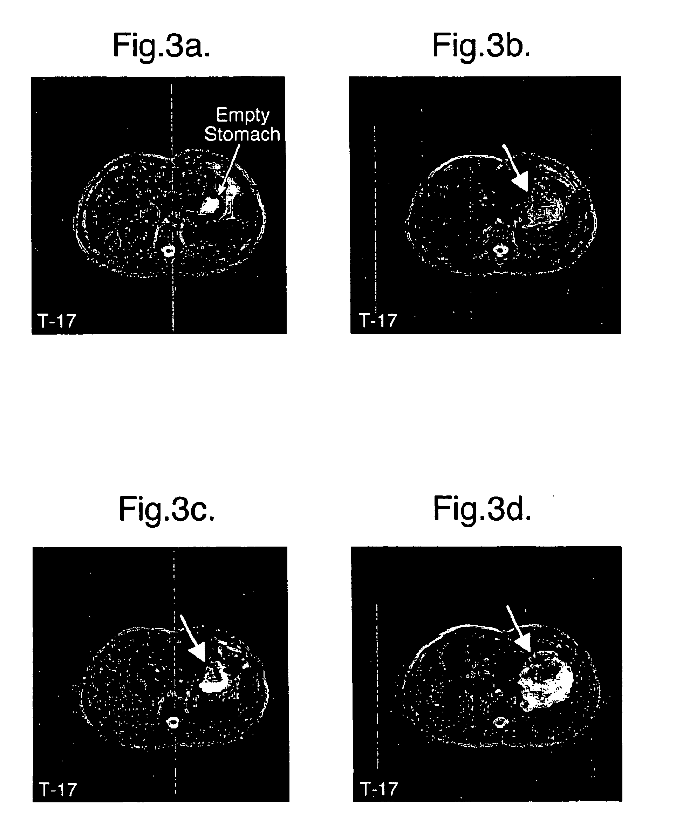

Figure 3 shows the respective MRI images of the stomach of the volunteer that ingested 250 ml of the composition.

From these images it was concluded that gel lumps of effective size formed unexpectedly quick both after ingestion of the 100 ml sample as well as after ingestion of the 250 ml sample. The finding that the 100 ml sample was already very effective was surprising. The gel lumps that had formed had sufficient strength to withstand shear of the stomach, such as to effectively provide a feeling of satiety for a satifactory period of time.

## Claims

1. Use of a shelf-stable liquid composition comprising, based on the total weight of the composition, a combination of:
i) 0.05 - 5.0 wt% of HM pectin;
ii) 0.1 - 5.0 wit% of alginate; and
iv) 50 - 99 wt% of water;
having a pH of greater than 3.6 and below 5.0, wherein the viscosity of the liquid composition is within the range of 0.003-1.0 Pa.s at a shear rate of 10 s⁻¹ and a temperature of 20 °C, said composition having a force to fracture of 0.65-12 N at a temperature of 37 °C and a *pH < 3.5,* said composition being consumed in a volume of 100 to 300 ml by a person for enhancing feelings of satiety and/or inducing satiation upon ingestion.

2. Use according to claim 1, wherein the total amount of the combination of the pectin and the alginate ranges from, 0.2 - 5.0 wt%.

3. Use of a shelf-stable liquid composition according to claim 1 or 2, wherein the alginate has a guluronate content of at least 60%.

4. Use of a shelf stable liquid composition according to any one of the preceding claims, excluding aqueous acidic protein suspensions comprising high methoxyl pectin and propylene glycol alginate in a combined weight of 10 to 70 % of the weight of the protein.

5. Use of a shelf-stable liquid composition according to any one of the preceding claims, wherein the pectin has a methyl ester content of at least 50 %.

6. Use of a shelf-stable liquid composition according to any one of the preceding claims, wherein the average molecular weight of the alginate ranges from 100-300 kDa.

7. Use of a shelf-stable liquid composition according to any one of the preceding claims, comprising fruit and/or vegetable solids in an amount ranging from 0.1 to 30 wt%.

8. Use of a shelf-stable liquid composition as defined in any one of the preceding claims, wherein the shelf-stable liquid composition does not contain any pharmacologically active substances selected from the goup of antibiotics, antibacterial agents and agents for treating and/or preventing gastritis,
stomach ulcers and/or duodenal ulcers.

9. Shelf-stable liquid composition comprising, based on the total weight of the composition, a combination of:
i) 0.05 - 5.0 wt% of HM pectin;
ii) 0.1 - 5.0 wt% of alginate; and
iv) 50 - 99 wit% of water;
having a pH of greater than 3.6 and below 5.0, wherein the viscosity of the liquid composition is within the range of 0.003-1.0 Pa.s at a shear rate of 10 s⁻¹ and a temperature of 20 °c, said composition having a force to fracture of 0.65-12 N at a temperature of 37 °C and a pH < 3.5, said composition being consumed in a volume of 100 to 300 ml by a person for use in the treatment of obesity, being overweight.

10. Shelf-stable liquid composition according to claim 9, for use in the prophylactic or curative treatment of obesity, being overweight, hypercholesterolemia, hyperlipidemia, hyperglycaemia, insulin resistance and/or metabolic syndrome in a mammal.

11. Shelf-stable liquid composition according to claim 9 or 10, wherein the total amount of the combination of the pectin and the alginate ranges from, 0.2 - 5.0 wt%.

12. Shelf-stable liquid composition according to claim 9 to 11, wherein the alginate has a guluronate content of at least 60%.

13. Shelf stable liquid composition according to any one of claims 9 to 12, excluding aqueous acidic protein suspensions comprising high methoxyl pectin and propylene glycol alginate in a combined weight of 10 to 70 % of the weight of the protein.

14. Shelf-stable liquid composition according to any one of claims 9 to 13, wherein the pectin has a methyl ester content of at least 50 %.

15. Shelf-stable liquid composition according to any one of claims 9 to 14, wherein the average molecular weight of the alginate ranges from 100-300 kDa.

16. Shelf-stable liquid composition according to any one of claims 9 to 15, comprising fruit and/or vegetable solids in an amount ranging from 0.1 to 30 wt%.

17. Shelf-stable liquid composition as defined in any one of claims 9 to 16, wherein the shelf-stable liquid composition does not contain any pharmacologically active substances selected from the goup of antibiotics, antibacterial agents and agents for treating and/or preventing gastritis, stomach ulcers and/or duodenal ulcers.

## Patentansprüche

1. Verwendung einer haltbaren flüssigen Zusammensetzung, die, bezogen auf das Gesamtgewicht der Zusammensetzung, eine Kombination von:
i) 0,05 - 5,0 Gew.-% HM-Pektin;
ii) 0,1 - 5,0 Gew.-% Alginat und
iv) 50 - 99 Gew.-% Wasser umfasst,
die einen pH von größer als 3,6 und kleiner als 5,0 hat, wobei die Viskosität der flüssigen Zusammensetzung innerhalb des Bereichs von 0,003 - 1,0 Pa.s bei einer Scherrate von 10 s⁻¹ und einer Temperatur von 20 °C liegt, wobei die Zusammensetzung eine Kraft bis zum Bruch von 0,65-12 N bei einer Temperatur von 37 °C und einem pH < 3,5 hat, wobei die Zusammensetzung in einem Volumen von 100 bis 300 ml von einer Person zur Verstärkung des Sättigungsgefühls und/oder zur Induzierung von Sättigung bei Aufnahme konsumiert wird.

2. Verwendung gemäß Anspruch 1, wobei die Gesamtmenge der Kombination des Pektins und des Alginats im Bereich von 0,2 - 5,0 Gew.-% liegt.

3. Verwendung einer haltbaren flüssigen Zusammensetzung gemäß Anspruch 1 oder 2, wobei das Alginat einen Guluronatgehalt von wenigstens 60 % hat.

4. Verwendung einer haltbaren flüssigen Zusammensetzung gemäß einem der vorangehenden Ansprüche, ausgenommen wässrige saure Proteinsuspensionen, umfassend Pektin mit hohem Methoxylgehalt und Propylenglykolalginat mit einem kombinierten Gewicht von 10 bis 70 % des Gewichts des Proteins.

5. Verwendung einer haltbaren flüssigen Zusammensetzung gemäß einem der vorangehenden Ansprüche, wobei das Pektin einen Methylestergehalt von wenigstens 50 % hat.

6. Verwendung einer haltbaren flüssigen Zusammensetzung gemäß einem der vorangehenden Ansprüche, wobei das durchschnittliche Molekulargewicht des Alginats im Bereich von 100-300 kDa liegt.

7. Verwendung einer haltbaren flüssigen Zusammensetzung gemäß einem der vorangehenden Ansprüche, die Frucht- und/oder Gemüsefeststoffe in einer Menge im Bereich von 0,1 bis 30 Gew.-% umfasst.

8. Verwendung einer haltbaren flüssigen Zusammensetzung, wie sie in einem der vorangehenden Ansprüche definiert ist, wobei die haltbare flüssige Zusammensetzung keine pharmakologisch aktive Substanzen, ausgewählt aus der Gruppe von Antibiotika, antibakteriellen Mitteln und Mitteln zur Behandlung und/oder Prävention von Gastritis, Magengeschwüren und/oder Duodenalgeschwüren, enthält.

9. Haltbare flüssige Zusammensetzung, die, bezogen auf das Gesamtgewicht der Zusammensetzung, eine Kombination von:
i) 0,05 - 5,0 Gew.-% HM-Pektin;
ii) 0,1 - 5,0 Gew.-% Alginat und
iv) 50 - 99 Gew.-% Wasser umfasst,
die einen pH von größer als 3,6 und kleiner als 5,0 hat, wobei die Viskosität der flüssigen Zusammensetzung bei einer Scherrate von 10 s⁻¹ und einer Temperatur von 20 °C im Bereich von 0,003-1,0 Pa.s liegt, wobei die Zusammensetzung eine Kraft bis zum Bruch von 0,65-12 N bei einer Temperatur von 37 °C und einem pH < 3,5 hat, wobei die Zusammensetzung in einem Volumen von 100 bis 300 ml von einer Person zur Verwendung bei der Behandlung von Adipositas, Übergewicht konsumiert wird.

10. Haltbare flüssige Zusammensetzung gemäß Anspruch 9 zur Verwendung bei der prophylaktischen oder heilenden Behandlung von Adipositas, Übergewicht, Hypercholesterinämie, Hyperlipidämie, Hyperglycämie, Insulinresistenz und/oder metabolischem Syndrom bei einem Säuger.

11. Haltbare flüssige Zusammensetzung gemäß Anspruch 9 oder 10, wobei die Gesamtmenge der Kombination des Pektins und des Alginats im Bereich von 0,2 - 5,0 Gew.-% liegt.

12. Haltbare flüssige Zusammensetzung gemäß Anspruch 9 bis 11, wobei das Alginat einen Guluronatgehalt von wenigstens 60 % hat.

13. Haltbare flüssige Zusammensetzung gemäß einem der Ansprüche 9 bis 12, ausgenommen wässrige saure Proteinsuspensionen, umfassend Pektin mit hohem Methoxylgehalt und Propylenglykolalginat mit einem kombinierten Gewicht von 10 bis 70 % des Gewichts des Proteins.

14. Haltbare flüssige Zusammensetzung gemäß einem der Ansprüche 9 bis 13, wobei das Pektin einen Methylestergehalt von wenigstens 50 % hat.

15. Haltbare flüssige Zusammensetzung gemäß einem der Ansprüche 9 bis 14, wobei das durchschnittliche Molekulargewicht des Alginats im Bereich von 100-300 kDa ist.

16. Haltbare flüssige Zusammensetzung gemäß einem der Ansprüche 9 bis 15, die Frucht- und/oder Gemüsefeststoffe in einer Menge im Bereich von 0,1 bis 30 Gew.-% umfasst.

17. Haltbare flüssige Zusammensetzung, wie sie in einem der Ansprüche 9 bis 16 definiert ist, wobei die haltbare flüssige Zusammensetzung keinerlei pharmakologisch aktive Substanzen, ausgewählt aus der Gruppe von Antibiotika, antibakteriellen Mitteln und Mitteln zur Behandlung und/oder Prävention von Gastritis, Magengeschwüren und/oder Duodenalgeschwüren, enthält.

## Revendications

1. Utilisation d'une composition liquide de longue conservation comprenant, sur la base du poids total de la composition, une combinaison de :
i) 0,05 % en poids à 5,0 % en poids de pectine HM ;
ii) 0,1 % en poids à 5,0 % en poids d'alginate ; et
iv) 50 % en poids à 99 % en poids d'eau ;
ayant un pH supérieur à 3,6 et inférieur à 5,0, dans laquelle la viscosité de la composition liquide se situe dans la plage allant de 0,003 Pa.s à 1,0 Pa.s à un taux de cisaillement de 10 s⁻¹ et à une température de 20°C, ladite composition présentant une force à la rupture de 0,65 N à 12 N à une température de 37°C et à un pH < 3,5, ladite composition étant consommée en un volume de 100 ml à 300 ml par une personne pour renforcer la sensation de satiété et/ou induire la satiété après son ingestion.

2. Utilisation selon la revendication 1, dans laquelle la quantité totale de la combinaison de la pectine et de l'alginate est située dans la plage allant de 0,2 % en poids à 5,0 % en poids.

3. Utilisation d'une composition liquide de longue conservation selon la revendication 1 ou 2, dans laquelle l'alginate présente une teneur en guluronate d'au moins 60 %.

4. Utilisation d'une composition liquide de longue conservation selon l'une quelconque des revendications précédentes, en excluant les suspensions aqueuses de protéines acides comprenant une pectine méthoxylée supérieure et de l'alginate de propylèneglycole en un poids combiné de 10 % à 70 % du poids des protéines.

5. Utilisation d'une composition liquide de longue conservation selon l'une quelconque des revendications précédentes, dans laquelle la pectine présente une teneur en ester de méthyle d'au moins 50 %.

6. Utilisation d'une composition liquide de longue conservation selon l'une quelconque des revendications précédentes, dans laquelle le poids moléculaire moyen de l'alginate est situé dans la plage allant de 100 kDa à 300 kDa.

7. Utilisation d'une composition liquide de longue conservation selon l'une quelconque des revendications précédentes, comprenant des matières sèches de fruits et/ou de légumes en une quantité allant de 0,1 % en poids à 30 % en poids.

8. Utilisation d'une composition liquide de longue conservation selon l'une quelconque des revendications précédentes, dans laquelle la composition liquide de longue conservation ne contient pas de substances actives sur le plan pharmacologique choisies dans le groupe des antibiotiques, des agents antibactériens et des agents destinés à traiter et/ou à prévenir la gastrite, les ulcères de l'estomac et/ou les ulcères duodénaux.

9. Composition liquide de longue conservation comprenant, sur la base du poids total de la composition, une combinaison de :
i) 0,05 % en poids à 5,0 % en poids de pectine HM ;
ii) 0,1 % en poids à 5,0 % en poids d'alginate ; et
iv) 50 % en poids à 99 % en poids d'eau ;
ayant un pH supérieur à 3,6 et inférieur à 5,0, dans laquelle la viscosité de la composition liquide se situe dans la plage allant de 0,003 Pa.s à 1,0 Pa.s à un taux de cisaillement de 10 s⁻¹ et à une température de 20°C, ladite composition présentant une force à la rupture de 0,65 N à 12 N à une température de 37°C et à un pH < 3,5, ladite composition étant consommée en un volume de 100 ml à 300 ml par une personne pour un usage dans le traitement de l'obésité et du surpoids.

10. Composition liquide de longue conservation selon la revendication 9, pour un usage dans le traitement prophylactique ou curatif de l'obésité, du surpoids, de l'hypercholestérolémie, de l'hyperlipidémie, de l'hyperglycémie, de la résistance à l'insuline et/ou du syndrome métabolique chez un mammifère.

11. Composition liquide de longue conservation selon la revendication 9 ou 10, dans laquelle la quantité totale de la combinaison de la pectine et de l'alginate est située dans la plage allant de 0,2 % en poids à 5,0 % en poids.

12. Composition liquide de longue conservation selon les revendications 9 à 11, dans laquelle l'alginate présente une teneur en guluronate d'au moins 60 %.

13. Composition liquide de longue conservation selon l'une quelconque des revendications 9 à 12, en excluant les suspensions aqueuses de protéines acides comprenant une pectine méthoxylée supérieure et de l'alginate de propylèneglycol en un poids combiné de 10 % à 70 % du poids des protéines.

14. Composition liquide de longue conservation selon l'une quelconque des revendications 9 à 13, dans laquelle la pectine présente une teneur en ester de méthyle d'au moins 50 %.

15. Composition liquide de longue conservation selon l'une quelconque des revendications 9 à 14, dans laquelle le poids moléculaire moyen de l'alginate est situé dans la plage allant de 100 kDa à 300 kDa.

16. Composition liquide de longue conservation selon l'une quelconque des revendications 9 à 15, comprenant des matières sèches de fruits et/ou de légumes en une quantité allant de 0,1 % en poids à 30 % en poids.

17. Composition liquide de longue conservation selon l'une quelconque des revendications 9 à 16, dans laquelle la composition liquide de longue conservation ne contient pas de substances actives sur le plan pharmacologique choisies dans le groupe des antibiotiques, des agents antibactériens et des agents destinés à traiter et/ou à prévenir la gastrite, les ulcères de l'estomac et/ou les ulcères duodénaux.
